# EUROPEAN PATENT APPLICATION

(11) **EP 2 277 439 A2**
(43) Date of publication of application: **26.01.2011**
(21) Application number: 09746727.8
(22) Date of filing: 29.04.2009
(51) Int. Cl.: A61B 5/01

(54) **PORTABLE CLINICAL THERMOMETER CAPABLE OF PROVIDING VISUAL IMAGES**

(30) Priority: 13.05.2008 KR 20080044145
(71) Applicant: Aram Huvis Co., Ltd., Gyeonggi-do 462-120 (KR); Eulji University Industry Cooperation, Gyeonggi-do 461-815 (KR)
(72) Inventor: PAK, Dong Sun, Yongin-si Gyeonggi-do 446-912 (KR); LEE, Woo Chel, Seongnam-si Gyeonggi-do 463-060 (KR)
(74) Representative: Barth, Stephan Manuel
(86) International application number: PCT/KR2009/002265
(87) International publication number: WO 2009/139548

(57) **Abstract**

The present invention relates to a portable clinical thermometer capable of providing visual images, and more particularly, to a portable clinical thermometer capable of providing visual images, which can take a body temperature, and simultaneously photographs a living body region where occurrence of a problem is suspected and then provides the photographed image such that any emergency can be handled easily.

A portable clinical thermometer capable of providing visual images according to the present invention comprises a body having an insert provided to protrude at one side thereof so that the insert is inserted into a living body; a clinical thermometer module provided at an inner distal end of the insert to detect infrared emitted from the living body and generate an electric signal corresponding to an intensity of the detected infrared; a camera module provided at the inner distal end of the insert to take an image of an interior of the living body and generate an image signal; a microprocessor for receiving the electric signal generated in the clinical thermometer module to calculate a body temperature and receiving the image signal generated in the camera module to generate an image; a memory for receiving and storing the body temperature information and the image information from the microprocessor; and a display for displaying the body temperature and the image respectively calculated and generated in the microprocessor, wherein the body temperature information and the image information are displayed when being measured, and also changes in body temperature and living body image with the passage of time are displayed using the body temperature information and the image information stored in the memory.

## Description

### BACKGROUND OF THE INVENTION

### 1. Field of the Invention

The present invention relates to a portable clinical thermometer capable of providing visual images, and more particularly, to a portable clinical thermometer capable of providing visual images, which can take a body temperature, and simultaneously photographs a living body region where occurrence of a problem is suspected and then provides the photographed image such that any emergency can be handled easily.

### 2. Description of the Related Art

When a human body is exposed to contagious bacteria or viruses, the immune system of the human body fights against them, so that a body temperature is increased to a certain level. Here, if the temperature is increased over 38.3°C, we say someone have a high fever. Though not catching a disease, a human body undergoes a procedure of overcoming pathogenic bacteria by means of the immune system, which can be learned by checking whether the temperature is changed, more accurately whether the temperature is increased over a certain level.

In order to measure a body temperature, conventionally, a mercury clinical thermometer was put into the armpit. However, this method takes a relatively long time for measuring the body temperature and does not allow exact temperature detection. Thus, an infrared clinical thermometer is recently put into the ear hole and detects the infrared generated from the eardrum to measure the temperature. The eardrum shares blood with the hypothalamus of the brain that controls the temperature, so that the measurement of temperature of the eardrum is the fastest and most accurate way for checking a change in temperature.

Infrared clinical thermometers are used for measuring a body temperature not only at hospitals but also at houses. In particular, children with a weak immune system frequently have fevers, so that the infrared clinical thermometer is substantially a necessity for homes with children.

Meanwhile, in a case where an ear, nose or throat disease occurs to a child who cannot yet express his/her intention very well, for example in a case where any alien substance enters the ear or nose or the ear or nose is inflamed, the child cries to appeal his/her pain, but his/her parents often worry since they do not know why the child is crying. In such a case, the parents try to check the inside of the ear or nose of the child, but it is not easy since the child struggles to refuse the parents' action. Thus, though the symptom is not serious, the parents should bring the child to the hospital so as to check the reason.

In addition, it is not easy even for an adult to directly check a disease occurring at a dark and narrow spot such as the inside of the ear or nose, so that the adult goes to hospital though the disease is not so serious, which consumes time and cost.

### SUMMARY OF THE INVENTION

The present invention is conceived to solve the aforementioned problems in the prior art. An object of the present invention is to provide a portable clinical thermometer capable of providing visual images, which has a camera module mounted at a distal end of an ear clinical thermometer commonly used in the art so as to take a body temperature and simultaneously take an image on a region that is suspected as having a disease so that any emergent situation can be easily handled.

A portable clinical thermometer capable of providing visual images according to the present invention comprises a body having an insert provided to protrude at one side thereof so that the insert is inserted into a living body; a clinical thermometer module provided at an inner distal end of the insert to detect infrared emitted from the living body and generate an electric signal corresponding to an intensity of the detected infrared; a camera module provided at the inner distal end of the insert to take an image of an interior of the living body and generate an image signal; a microprocessor for receiving the electric signal generated in the clinical thermometer module to calculate a body temperature and receiving the image signal generated in the camera module to generate an image; a memory for receiving and storing the body temperature information and the image information from the microprocessor; and a display for displaying the body temperature and the image respectively calculated and generated in the microprocessor, wherein the body temperature information and the image information are displayed when being measured, and also changes in body temperature and living body image with the passage of time are displayed using the body temperature information and the image information stored in the memory.

A portable clinical thermometer capable of providing visual images according to the present invention can be carried personally to take a body temperature, and simultaneously, a user of the clinical thermometer can obtain an inner image of the living body with a disease to primarily check occurrence or not of symptom, so that any emergent situation can be easily handled. In addition, the portable clinical thermometer capable of providing visual images according to the present invention has a camera module mounted at a distal end of an insert such that the camera module can closely approach an interior of the living body, thereby ensuring a wider visual range and obtaining a more accurate and detailed image.

### BRIEF DESCRIPTION OF THE DRAWINGS

Fig. 1 is a side view showing a portable clinical thermometer capable of providing visual images according to the present invention;
Fig. 2 is a front view showing the portable clinical thermometer capable of providing visual images according to the present invention;
Fig. 3 is a rear view showing the portable clinical thermometer capable of providing visual images according to the present invention;
Fig. 4 is a sectional view showing the inner configuration of an insert of the portable clinical thermometer capable of providing visual images according to the present invention; and
Fig. 5 is a block diagram showing the inner configuration of the portable clinical thermometer capable of providing visual images according to the present invention.

### DESCRIPTION OF THE PREFERRED EMBODIMENT

Hereinafter, although preferred embodiments of the present invention will be described in detail, the present invention dose not limited to the following embodiments as long as they do not depart from the spirit of the invention.

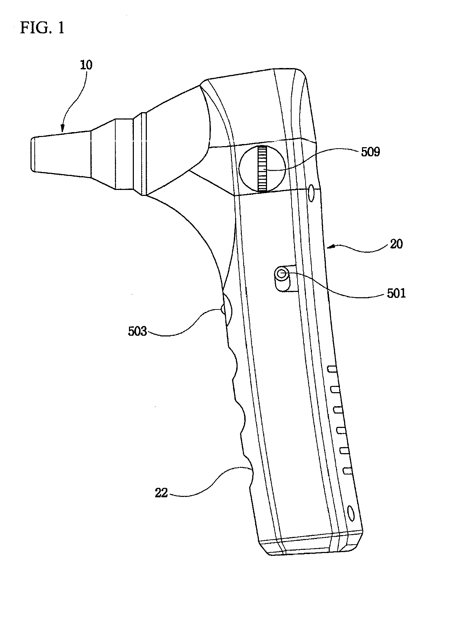

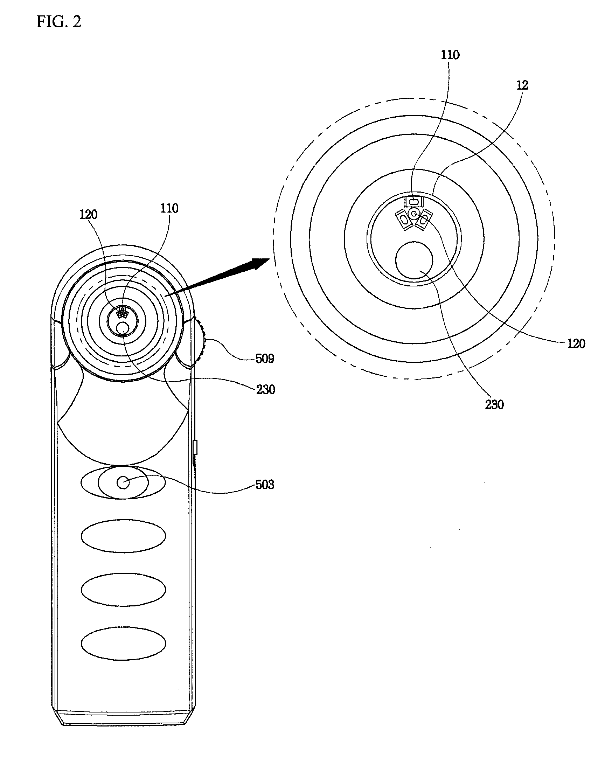

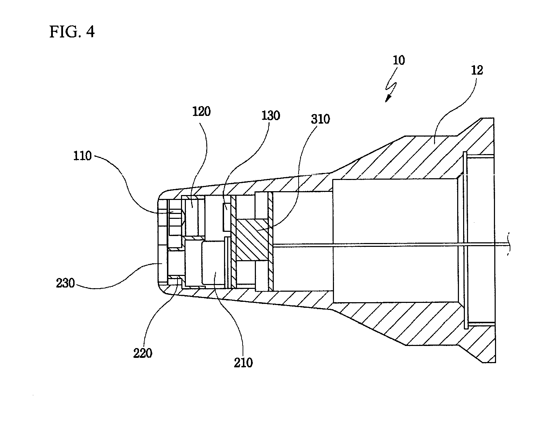

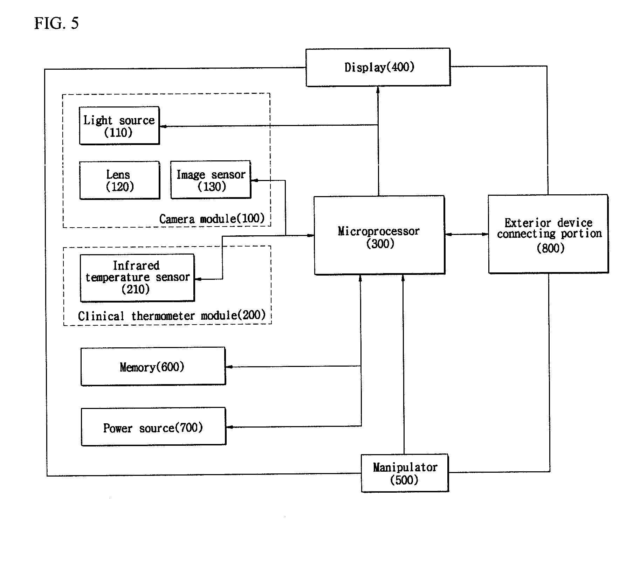

Figs. 1 to 3 show the outer configuration of a portable clinical thermometer capable of providing visual images according to the present invention, Fig. 4 is a sectional view showing the inner configuration of an insert of the portable clinical thermometer capable of providing visual images according to the present invention, and Fig. 5 is a block diagram showing the inner configuration of the portable clinical thermometer capable of providing visual images according to the present invention. The present invention will be described with reference to the accompanying drawings.

A portable clinical thermometer capable of providing visual images according to the present invention is a device that is inserted into the external auditory canal reaching from an entrance of the ear to an eardrum to measure a body temperature and simultaneously obtains and displays an image of a narrow and dark interior of the living body such as an oral or nasal cavity. Also, the portable clinical thermometer allows a user to frequently take and store an image of a specific living body region and then check how the living body region is changed.

Referring to the figures, the portable clinical thermometer capable of providing visual images according to the present invention includes a body 20 having an insert 10 formed in the shape of a protrusion to be inserted into a living body such as the ear, a clinical thermometer module 200 provided in the insert 10 to take a body temperature of the living body, a camera module 100 provided in the insert 10 to take an image of the living body, a microprocessor 300 provided in the body 20 to calculate a body temperature and generate a living body image, and a display provided out of the body 20 to display the calculated body temperature and the living body image.

A plurality of gripping grooves 22 or an uneven portion is formed at a lower portion of the body 20 for easy grip. Also, a plurality of manipulators 500 for manipulating the camera module 100, the clinical thermometer module 200 and the microprocessor 300 are formed on front, side and rear surfaces of the body 20 to select and execute various functions such as a power button 501 for turning the thermometer on/off, a temperature measurement button 505 for starting/stopping the temperature measurement operation, a photographing selection button 503 for taking an image of the living body, a memory button 507 for storing the measured body temperature or the photographed image, and the like.

The manipulators 500 comprise at least three buttons for power supply, temperature measurement and photographing, and are configured to turn the device on/off or select starting/stopping/storing/deleting the temperature measurement/photographing at each pressing. Also, a wheel 509 provided at a side of the body 20 has a scrolling function so as to read plural data stored in a memory 600 and a zooming-in/out function for an image that is already photographed or is being photographed.

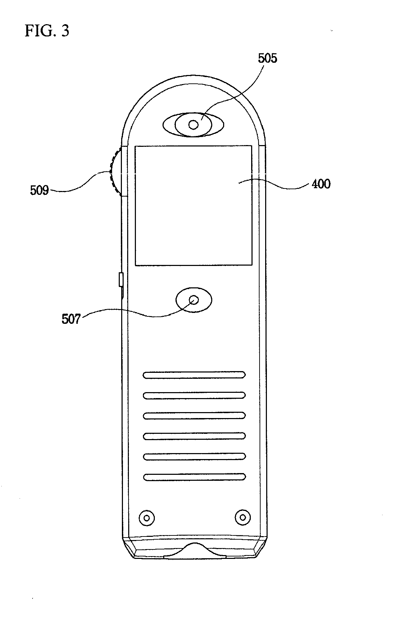

Referring to Fig. 3, the display 400 formed on an upper portion of the rear surface of the body 20 may be configured with a full-color LCD (Liquid Crystal Display) or an organic EL (Electroluminescence) display so as to allow exact determination on a living body state. Also, the display 400 displays a measured body temperature while outputting an image of the living body and may also display a battery residual capacity of the device, an error message, an operation state, and the like.

Referring to Figs. 4 and 5, an outer portion of the insert 10 formed in the top of the body 20 is provided with a speculum 12 having a cavity and a conical shape with a diameter gradually decreased toward a distal end of the insert 10 so as to ensure easier insertion of the insert 10 into a narrow living body region such as the oral or nasal cavity and allow easier incidence of infrared generated from the living body and the light reflecting from the living body. At an inner distal end of the speculum 12, there are provided the clinical thermometer module 200 for taking a body temperature and the camera module 100 for taking an image of the living body.

The speculum 12 may be configured to be fixedly or detachably mounted to the body 20 and have the open distal end for making temperature measurement and photographing easy. In addition, the speculum 12 may be made of a material that is soft so as not to give a feeling of irritation when the speculum 12 is inserted into and directly contacted with the oral or nasal cavity and is endurable not to be deformed or denatured by frequent disinfections using chemicals.

The camera module 100 includes a lens 120 provided at the distal end of the insert 10, a light source 110 mounted around the lens 120 to emit light into the living body, and an image sensor 130 provided at the rear of the lens 120 to receive the light passing through the lens 120 and generate an image.

The lens 120 is fixed at the front of the image sensor 130 and condenses the light incident from the outside toward the image sensor 130 so as to take a clear image.

The light source 110 comprises at least one LED (Light Emitting Diode) and is mounted around the lens 120 to emit light to a narrow or dark living body interior such as the oral or nasal cavity so as to allow generating a clear image under a condition of a relatively small intensity of illumination.

In addition, the image sensor 130 of the camera module 100, which may adopt CCD (Charged Coupled Device) or CMOS (Complementary MOS), receives the light passing through the lens 120 or the light reflecting from the living body to generate image signals.

The camera module 100 so configured is provided at the distal end of the insert 10 and thus ensures a relatively wide visual range, so that it is possible to take an image over a wider region, and for example, the camera module 100 allows a user to check a state of not only a diseased part but also surrounding regions thereof.

The clinical thermometer module 200 is provided in a hollow housing 220 and may be separated from the camera module 100 by the hollow housing 220. The housing 220 is used as a passage through which infrared emitted from the eardrum may pass, and the housing may be made of a metal material with high reflectivity and high thermal conductivity so as to minimize the loss of infrared.

The clinical thermometer module 200 includes the housing 220, a sealing member 230 provided at a distal end of the housing 220 to seal the inside of the housing 220, and an infrared temperature sensor 210 for detecting infrared from the eardrum to generate electric signals.

The sealing member 230 is used for preventing the interior of the housing 220 from being contaminated and at the same time minimizing the loss of infrared. Silicon wafer may be used as the sealing member 230.

The infrared temperature sensor 210 detects infrared and converts it into heat, and then measures temperature of the converted heat to generate electric signals corresponding to the measured temperature.

The light source 110 and the image sensor 130 of the camera module 100 and the infrared temperature sensor 210 of the clinical thermometer module 200 may be mounted to a single printed circuit board 310, which may greatly decrease a volume of the device.

A microprocessor 300 for controlling the operation of the respective components, such as the camera module 100 and the clinical thermometer module 200, and receiving the signals generated in the camera module 100 or the clinical thermometer module 200 to generate an image or calculate a body temperature is mounted to the printed circuit board 310.

Also, the portable clinical thermometer capable of providing visual images according to the present invention is provided with a memory 600 for storing the body temperatures measured in the clinical thermometer module 200 and the living body images taken by the camera module 100 and a power source 700 for supplying power to the respective components. In this configuration, the body temperatures or the living body images subsequently stored in the memory 600 may be read by the microprocessor 300 in accordance with a user's request inputted by the manipulator 500 such as the wheel 509 and then outputted through the display 400 to show a change in body temperature or living body image with the passage of time. The portable clinical thermometer capable of providing visual images according to the present invention may be used with a disposable or rechargeable battery or in connection with an electric outlet.

Also, an external device connecting portion 800 for the connection of an external device to the microprocessor 300 is formed in the body 20 so that the information stored in the memory 600 may be transferred to a portable memory such as a USB memory or SD (Secure Digital) card. In addition, the information stored in the memory 600 may be outputted to a large screen such as a television while the living body is photographed, so that it is possible to check the state of the living body more exactly.

The portable clinical thermometer capable of providing visual images according to the present invention can be carried personally to measure a body temperature and obtain an inner image of the living body with a disease, which allows primary checking on the living body, so that the present invention may be distributed to individuals and medical institutions and promote the medical industry.

Although the present invention has been described and illustrated in connection with the specific embodiments as described above, it will be readily understood that various modifications can be made thereto without departing from the scope of the present invention. Therefore, the scope of the present invention is not limited to the embodiments described above but is defined by the appended claims and the equivalents thereto.

## Claims

1. A portable clinical thermometer capable of providing visual images, comprising:
a body having an insert provided to protrude at one side thereof so that the insert is inserted into a living body;
a clinical thermometer module provided at an inner distal end of the insert to detect infrared emitted from the living body and generate an electric signal corresponding to an intensity of the detected infrared;
a camera module provided at the inner distal end of the insert to take an image of an interior of the living body and generate an image signal;
a microprocessor for receiving the electric signal generated in the clinical thermometer module to calculate a body temperature and receiving the image signal generated in the camera module to generate an image;
a memory for receiving and storing the body temperature information and the image information from the microprocessor; and
a display for displaying the body temperature and the image respectively calculated and generated in the microprocessor,
wherein the body temperature information and the image information are displayed when being measured, and also changes in body temperature and living body image with the passage of time are displayed using the body temperature information and the image information stored in the memory.

2. The portable clinical thermometer as claimed in claim 1, wherein the clinical thermometer module includes:
a housing;
a sealing member for sealing a distal end of the housing; and
an infrared temperature sensor provided in the housing.

3. The portable clinical thermometer as claimed in claim 1, wherein the camera module includes:
a lens provided at the distal end of the insert for allow light incident from the living body to pass therethrough;
a light source provided around the lens to emit light into the living body; and
an image sensor provided at the rear of the lens.

4. The portable clinical thermometer as claimed in claim 3, wherein the light source comprises an LED (Light Emitting Diode).

5. The portable clinical thermometer as claimed in claim 1, wherein the display comprises an LCD (Liquid Crystal Display) or organic EL (Electroluminescence) display.

6. The portable clinical thermometer as claimed in claim 1, wherein the body is provided with a manipulator for manipulating the operation of the camera module, the clinical thermometer module and the microprocessor.

7. The portable clinical thermometer as claimed in claim 1, wherein the body is provided with an external device connecting portion for connecting an external device to the microprocessor.
